# EUROPEAN PATENT APPLICATION

(11) **EP 4 653 549 A1**
(43) Date of publication of application: **26.11.2025**
(21) Application number: 24876150.4
(22) Date of filing: 11.07.2024
(51) Int. Cl.: C12Q 1/6888, C12Q 1/6851, C12N 15/11

(54) **PROBE METHOD-BASED KIT AND METHOD FOR TRACKING BEHAVIOR TRAJECTORY OF ACIPENSER DABRYANUS DUMERIL RELEASED UPSTREAM OF YANGTZE RIVER**

(30) Priority: 08.10.2023 CN 202311293132
(71) Applicant: Chinese Sturgeon Research Institute of CTG, Yichang, Hubei 443100 (CN)
(72) Inventor: LI, Sha, Yichang, Hubei 443100 (CN); DANG, Yingchao, Yichang, Hubei 443100 (CN); LI, Zhiyuan, Yichang, Hubei 443100 (CN); JIANG, Wei, Yichang, Hubei 443100 (CN); YU, Zhaoxi, Yichang, Hubei 443100 (CN); LIU, Xueqing, Yichang, Hubei 443100 (CN); SU, Wei, Yichang, Hubei 443100 (CN)
(74) Representative: Keller Schneider Patent- und Markenanwälte AG
(86) International application number: PCT/CN2024/105009
(87) International publication number: WO 2025/077329

(57) **Abstract**

Disclosed are a probe method-based kit and method for tracking a behavior trajectory of Acipenser dabryanus Dumeril released upstream of the Yangtze River. By analyzing mitochondrial DNA sequences of Acipenser dabryanus Dumeril and common fish species in the Yangtze River basin, a pair of specific primers ND5-F4/ND5-R4 capable of specifically amplifying mitochondrial DNAs of Acipenser dabryanus Dumeril and a Taqman fluorescent probe ND5 Probe 4 are designed and screened. A kit composed of the specific primers, the Taqman fluorescent probe, and a ddPCR master mix is used to carry out ddPCR testing on environmental DNA samples collected before and after release, so that a behavior trajectory of the released Acipenser dabryanus Dumeril can be tracked and monitored, achieving "non-contact" tracking of the behavior trajectory of the released Acipenser dabryanus Dumeril, avoiding damage to fish bodies in the tracking process, leading to reduced tracking costs and improved accuracy of overall tracking.

## Description

### CROSS-REFERENCE TO RELATED APPLICATIONS

The present application claims priority to the Chinese patent application No. 202311293132.3, filed on October 8, 2023 to the China Patent Office and entitled "PROBE METHOD-BASED KIT AND METHOD FOR TRACKING BEHAVIOR TRAJECTORY OF ACIPENSER DABRYANUS DUMERIL RELEASED UPSTREAM OF YANGTZE RIVER", the entire content of which is incorporated herein by reference.

### TECHNICAL FIELD

The present application relates to the technical field of monitoring and tracking of fish species, in particular to a probe method-based kit and method for tracking a behavior trajectory of acipenser dabryanus dumeril released upstream of the Yangtze river.

### BACKGROUND

Acipenser dabryanus Dumeril, also called sturgeon or river sturgeon, is a kind of freshwater settled fishes, inhabits fast rivers with stone riverbed upstream of Yangtze river and is sturgeon living in the Yangtze river together with paddlefish and Chinese sturgeon. Compared to the other two kinds of sturgeon, the Acipenser dabryanus dumeril has a small body and a small weight but is faced with the same difficult situation, namely, the population size gets smaller and smaller. The international union for conservation of nature and natural resources (IUCN) has published an update report of a red list of global endangered species on July 21, 2022 and announced that paddlefish becomes extinct, acipenser dabryanus dumeril is extinct in the wild and the Chinese sturgeon still remains a "critically endangered" level. Wei Qiwei, the only one Chinese member in an IUCN sturgeon expert group and a chief scientist of the Chinese academy of fishery sciences stated that fortunately compared to the paddlefish, artificial conservation for the acipenser dabryanus dumeril and the Chinese sturgeon is achieved. For conserving the population quantity of wild acipenser dabryanus dumeril, the local government and relevant research institutions have conducted wild propagation and release activities for acipenser dabryanus dumeril every year since 2018, but up to now, fries of the acipenser dabryanus dumeril that are naturally propagated are not discovered throughout the whole Yangtze river. Whether the released acipenser dabryanus dumeril can have a stable population in the Yangtze river for natural propagation is the key to recovery of the population quantity of the wild acipenser dabryanus dumeril. The research on the behavior trajectory of the released acipenser dabryanus dumeril may help us to further study a behavior trend of the acipenser dabryanus dumeril in a natural water body, which thus lays the foundation of the study on recovery of the population of the acipenser dabryanus dumeril such as deducing a preferred habitat of the acipenser dabryanus dumeril and later recovery of the habitat.

A conventional method for testing fish species is to investigate a distribution range of the fish species by fishing through a fishing net, this method is harmful to a fish population though it is direct and effective and is laborious and time consuming. There may be the following main technologies for releasing, marking and tracking the acipenser dabryanus dumeril at present: a PIT marker, a sonar marker and a T-shaped marker. The sonar marker may implement recording of an activity process of the acipenser dabryanus dumeril in the Yangtze river but needs to arrange a plurality of sonar signal receivers along the river, which is costly. The PIT marker, the T-shaped marker and a DNA marker need to put a marker in a fish body, marked fish bodies account for a small proportion of released fish bodies, when deducing the behavior trajectory of a whole fish population by tracking the marked fish bodies, omission of testing frequently occurs, and there is very little collected available data, which is not conducive to fully studying behaviors, distribution preference and the like of the released fish bodies. Besides, the marking method is harmful to the fish bodies, a sonar unit may interfere with information receiving and sending of aquatic organisms, and consequently, the aquatic organisms have abnormal activities and die in severe case. Thus, it is necessary to develop a tracking method for revealing the behavior trajectory of the released acipenser dabryanus dumeril, which is not harmful to the fish bodies and does not affect normal life activities of the other aquatic organisms.

### SUMMARY OF THE INVENTION

For solving the above technical problems, the present application provides a probe method-based kit and method for tracking a behavior trajectory of acipenser dabryanus dumeril released upstream of the Yangtze river, and by arranging reasonable sampling points, collecting DNA in a water body environment, and then carrying out concentration testing on the collected DNA in the water body environment through design of a pair of specific primers and a Taqman fluorescent probe, "non-contact" track for the behavior trajectory of the released acipenser dabryanus dumeril is implemented.

For achieving the above objective, the present application provides a probe method-based kit for tracking a behavior trajectory of acipenser dabryanus dumeril released upstream of the Yangtze river, and the kit includes a pair of specific primers, a Taqman fluorescent probe, a DNA template, a ddPCR master mix and ddH₂O.

Preferably, the pair of specific primers includes an upstream primer ND5-F4 with a sequence of SEQ ID NO: 1; and a downstream primer ND5-R4 with a sequence of SEQ ID NO:2; and the Taqman fluorescent probe has a sequence of SEQ ID NO:3.

A 5' terminal of the Taqman fluorescent probe is connected with a fluorophore FAM, and a 3' terminal is connected with a quencher BHQ.

Preferably, the ddPCR master mix includes 2×supermix.

Preferably, the DNA template is a water body sample DNA, and the sample includes an acipenser dabryanus dumeril DNA concentration ≥0.05copies/µL.

The present application further provides a method for tracking a behavior trajectory of acipenser dabryanus dumeril released upstream of the Yangtze river, including the following steps:
(1) setting sampling sections in a researched water area, setting sampling points on the sampling sections, and collecting 2 L of water body on each sampling point every day one day before release and within five days after release;
(2) performing vacuum filtration on the collected water body within 24 h by using a mixed cellulose filter membrane, and preserving the mixed cellulose filter membrane obtained after filtration;
(3) extracting DNA on the mixed cellulose filter membrane and preserving the DNA at -20°C for standby use; and
(4) using the DNA obtained in step (3) as a template, carrying out ddPCR testing by using the tracking kit, determining a concentration of a target sequence in each collected water body, and thus determining the behavior trajectory of acipenser dabryanus dumeril.

Preferably, in step (1), spacing between the sampling sections is 2 km to 3 km, and the number of the sampling points is 2 to 5.

Preferably, in step (2), the mixed cellulose filter membrane has a pore diameter of 0.22 µm.

Preferably, in step (2), the preserving method is to perform preservation at -20°C or preserve in a Longmire's buffer at a room temperature.

Preferably, in step (3), a method for extracting the DNA is to perform extracting by using a Water DNA Kit (Omega).

Preferably, in step (4), an amplification system of the ddPCR testing is 8.5 µL of the DNA template, 0.5 µL of a reverse primer and 0.5 µL of a forward primer, 0.5 µL of the probe, and 10 µL of 2×ddPCR master mix; and an amplification procedure is initial denaturation for 10 min at 95°C; denaturation for 30s at 94°C, annealing for 40s at 60°C, extension for 30s at 72°C, and 40 cycles; and enzyme inactivation for 10 min at 98°C, and maintaining at 4°C.

The present application has the beneficial effects as follows.
1, By analyzing mitochondrial DNA sequences of the acipenser dabryanus dumeril and common fish species in a Yangtze River basin, the pair of specific primers ND5-F4/ND5-R4 and the Taqman fluorescent probe ND5 Probe4 able to specifically detect a target DNA fragment of acipenser dabryanus dumeril are designed and screened, and the target DNA fragment of the acipenser dabryanus dumeril may be specifically, stably and effectively amplified by using the pair of specific primers and the Taqman fluorescent probe.
2, By using the screened pair of specific primers ND5-F4/ND5-R4 and the Taqman fluorescent probe ND5Probe4, a concentration of the target DNA fragment of the acipenser dabryanus dumeril in an aquatic water body and a release water body may be detected successfully, though the concentration of the DNA of the acipenser dabryanus dumeril in the water sample is low, the ddPCR technology can still be used for testing as long as the concentration is ≥0.05copies/µL, and sensitivity is high.
3, The method for tracking the behavior trajectory of the acipenser dabryanus dumeril artificially propagated and released is provided, the concentration of the DNA in the water environment is detected based mainly on the screened pair of specific primers ND5-F4/ND5-R4 and Taqman fluorescent probe ND5 Probe4, so that a main position and the behavior trajectory of the released acipenser dabryanus dumeril are determined, compared with a conventional fishing method and common sonar monitoring method, the method is "non-contact" tracking, is not harmful to the fish bodies and saves time and effort, meanwhile, a large quantity of expensive devices are not needed, omission of testing caused by the limiter number of markers and arranged devices is avoided, and thus the behavior trajectory of the target fish species can be known comprehensively. As a whole, the probe method-based method for tracking the behavior trajectory of the acipenser dabryanus dumeril released upstream of the Yangtze river is not harmful to original aquatic organisms and target fish species in an investigated water area, not only is the behavior trajectory of marked fish bodies focused, but also the behavior trajectory of the entire population of the acipenser dabryanus dumeril released may be further tracked, and a research result is more representative than individual marker tracking.

### BRIEF DESCRIPTION OF THE DRAWINGS

Fig. 1 is a fluorescent quantitative amplification curve of various primer pairs and probes thereof in Example 1.
Fig. 2 is a correlation curve of a dilution multiple and ddPCR detection sensitivity in Example 3.
Fig. 3 is a concentration of environmental DNA of acipenser dabryanus dumeril in each section one day (October 23) before release in Example 4.
Fig. 4 is a concentration of environmental DNA of acipenser dabryanus dumeril in each section on the first day (October 25) after release in Example 4.
Fig. 5 is a concentration of environmental DNA of acipenser dabryanus dumeril in each section on the second day (October 26) after release in Example 4.
Fig. 6 is a concentration of environmental DNA of acipenser dabryanus dumeril in each section on the third day (October 27) after release in Example 4.
Fig. 7 is a concentration of environmental DNA of acipenser dabryanus dumeril in each section on the fourth day (October 28) after release in Example 4.
Fig. 8 is a concentration of environmental DNA of acipenser dabryanus dumeril in each section on the fifth day (October 29) after release in Example 4.
Fig. 9 is a diagram of position distribution of sonar signal fixed test stations in Example 5.

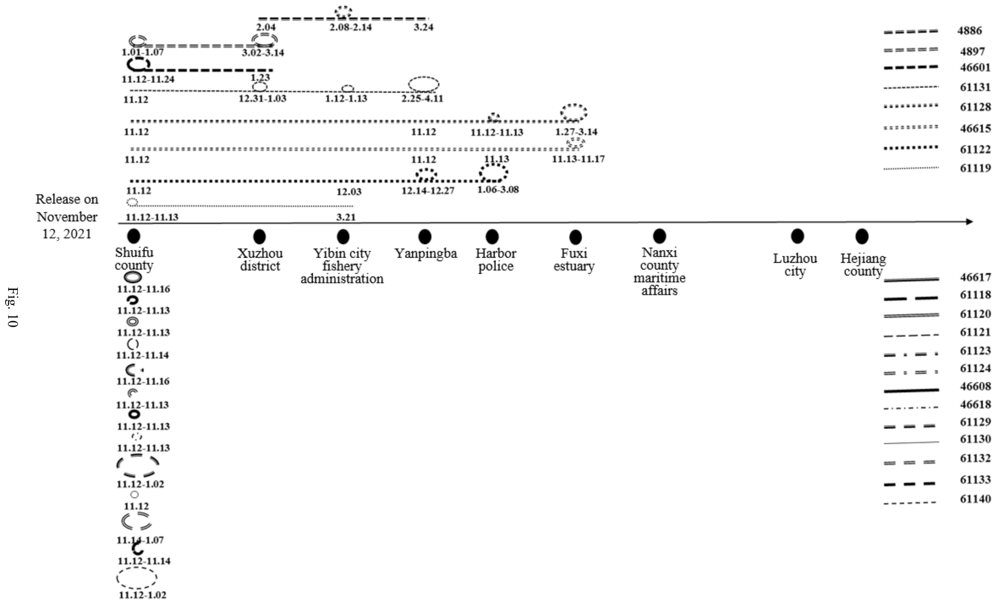

Fig. 10 is a diagram of sonar signal position distribution of acipenser dabryanus dumeril released in 2021 in Example 5; wherein a red solid circle represents a sonar signal fixed monitoring station, namely, a sonar monitoring section; line segments in different colors represent different individuals of the acipenser dabryanus dumeril marked by sonar; and an empty circle represents a frequency of occurrence of this acipenser dabryanus dumeril in a certain monitoring section, and a greater circle indicates the larger number of times that this acipenser dabryanus dumeril is monitored in this station and the longer duration of stay.

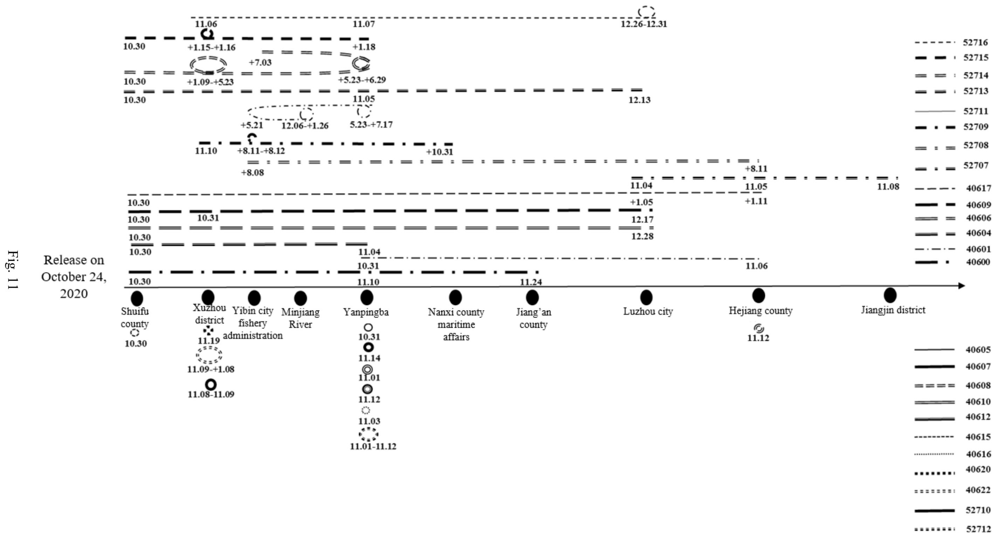

Fig. 11 is a diagram of sonar signal position distribution of acipenser dabryanus dumeril released in 2020 in Example 5; wherein a red solid circle represents a sonar signal fixed monitoring station, namely, a sonar monitoring section; line segments in different colors represent different individuals of acipenser dabryanus dumeril marked by sonar; and an empty circle represents a frequency of occurrence of this acipenser dabryanus dumeril in a certain monitoring section, and a greater circle indicates the larger number of times that this acipenser dabryanus dumeril is monitored in this station and the longer duration of stay.

### DETAILED DESCRIPTION

Technical solutions of the present application are further explained below with reference to accompanying drawings and specific examples. It is worth noting that the following examples are only preferred examples of the present application but are not construed as a limitation on the present application, and the protection scope of the present application is subject to the contents recorded in the claims. Modifications and replacements on the technical solutions of the present application by those of skill in the art without making creative efforts fall within the protection scope of the present application.

An E.Z.N.A.^{®}Water DNA Kit is purchased from an Omega company.

A ddPCR master mix is purchased from a Biorad company.

### Example 1 Design and screen of specific primers

According to a mitochondrial sequence of acipenser dabryanus dumeril, a sequence position with a larger difference is determined through a software comparison, a pair of primers and a probe are designed, then testing is performed by using a real-time fluorescent quantitative PCR technology, and the designed pair of primers and probe are screened, which specifically includes the following steps.
(1) According to a directory of recorded fish species from upper Yangtze river national nature reserve, a mitochondrial sequence of representative fish species is downloaded from each subfamily, there is a total of mitochondrial sequences of 35 fish species, through a DNAMAN software comparison, 7 pairs of specific primers are designed in sequence positions of 16S, COI and ND5 sites with a larger difference, and one probe is designed in a primer pair sequence interval, which are shown in Table 1 for details.
(2) Common fish species of cyprinidae, bagridae, cobitidae and catostomidae upstream of the Yangtze river are selected, fin rays or muscular tissue is obtained by clipping, fish body DNA is extracted, then testing is performed by using qubit, various fish bodies have DNA concentrations respectively: 59.4ng/µL for Chinese sucker; 23.2ng/pL for bonito; 25ng/µL for barbel steed; 21.8ng/µL for squaliobarbus curriculus; 110ng/µL for sarcocheilichthys parvus; 34.4ng/µL for ratmouth barbel; 42.4ng/µL for largemouth bronze gudgeon; 34.5ng/µL for bronze gudgeon; 26.2ng/µL for barred loach; 36.6ng/µL for rosy bitterling; 77.8ng/µL for ancherythroculter nigrocauda; 63.2ng/µL for mud fish; 120ng/µL for carp; 38.8ng/µL for liobagrus marginatus; 248ng/µL for Chinese lizard gudgeon; 45.8ng/µL for yellow catfish; 60.4ng/µL for spinibarbus sinensis; 20.4ng/µL for longsnout catfish; and 90.1ng/µL for acipenser dabryanus dumeril.

**Table 1 Primer and probe sequence**

| Primer and probe | Sequence |
|---|---|
| 16S-F1 | 5'-CTCACCACATACATCACAAAT G-3' |
| 16s-R1 | 5'-TCTCTATTGCGCTGCTTTGT-3' |
| 16S probel | 5'-CCATTTAATACCCCCAGATAG-3' |
| COI-F1 | 5'-TGGCACCTGATCCAAAATCCACT-3' |
| COI-R1 | 5'-GATTAGTGAGCCGATTGAGGAGA-3' |
| COI probel | 5'-AACACTTCCTAGGCCTCGCA-3' |
| ND5-F1 | 5'-AAGGCATAGAAGTCATCACCA-3' |
| ND5-R1 | 5'-GAGGCAAATTCTAGGATTGATC-3' |
| ND5 probel | 5'-GCCACCTTTGACATTAACATC-3' |
| ND5-F2 | 5'-TATTCGCCACCTCCCAAGGTA-3' |
| ND5-R2 | 5'-CAAGTTGTCAGGGCGACCT-3' |
| ND5 probe2 | 5'-TGGGTTTAATCCTAGCTGCCA-3' |
| ND5-F3 | 5'-ATCACCCCCACAATCCCACTA-3' |
| ND5-R3 | 5'-GGTGAGTTGCTATAGTTTGTC-3' |
| ND5 Probe3 | 5'-CTTCCCATCAATTATCCATCG-3' |
| ND5-F4 | 5'-GGTCGCCCTGACAACTTGC-3'(SEQ ID NO:1) |
| ND5-R4 | 5'-AGTGAGGGGGAGCATGGTGT-3'(SEQ ID NO:2) |
| ND5 Probe4 | 5'FAM-ACATCCAGCCAACTAGGCCTAATGAT-BHQ3'(SEQ ID NO:3) |
| ND5-F5 | 5'-CGAGTGGGAGACATTGGA-3' |
| ND5-R5 | 5'-GGAGACGGATGAGTAGGA-3' |
| ND5 Probe5 | 5'-TTCGCCACCTCCCAAGGTAG-3" |

(3) Real-time fluorescent quantitative PCR testing is performed on the fish body DNA obtained in step (2) respectively by using the pairs of primers and probe designed in step (1), and a pair of primers and a probe able to successfully amplify a target sequence are screened and determined; an amplification system is 20 µL: 10 µL of 2×SuperReal Premix, 0.6 µL of a forward primer and 0.6µL of a reverse primer, 0.4 µL of a probe, 0.6 µL of a DNA template, and 6.8 µL of ddH₂O; a reaction condition is: initial denaturation for 15 min at 95°C; denaturation for 1 s at 95°C; annealing/extension for 30 s at 60°C; and 40 cycles.

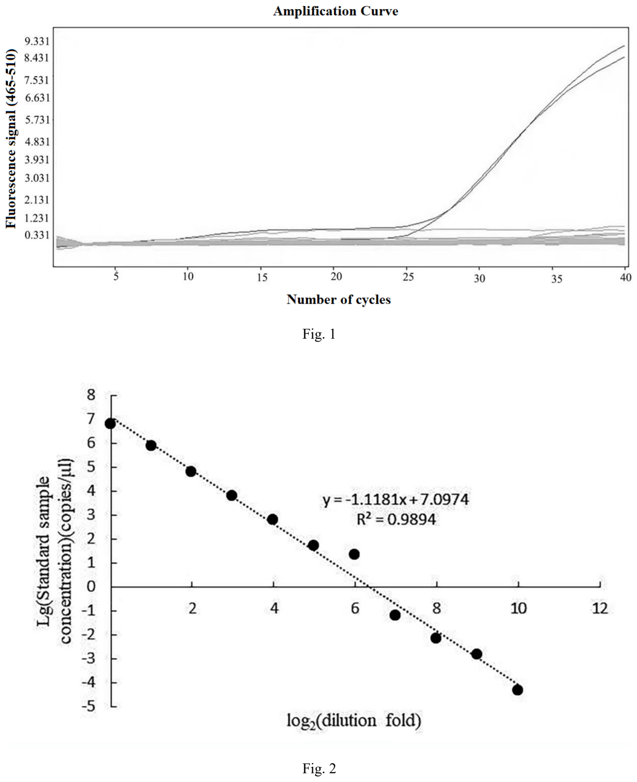

Results are shown in Fig. 1 that the designed pair of primers ND5-F4/ND5-R4 and probe ND5Probe4 thereof have high specificity. A fluorescent quantitative PCR amplification curve shows that no target sequence is obtained by amplification from DNA of other fish species except the acipenser dabryanus dumeril, and a combination of the pair of primers and probe may successfully separate the acipenser dabryanus dumeril from the other fish species.

### Example 2 Validation of effectiveness for water sample testing through pairs of primers and probe

An aquatic water body for the acipenser dabryanus dumeril is collected, ddPCR testing is performed by using the pair of specific primers and the probe screened in Example 1, whether testing may be successfully performed by using the primers and the probe through a water sample is determined, testing effectiveness is validated, 10-times dilution is performed on water sample DNA, and stability of the primers and the probe is preliminarily validated. The following steps are specifically included.
(1) 2 L of a water sample is collected in a culture pond which has a diameter of 4 m, a height of 1 m and a culture size of 30 cm and has about 100 pieces of acipenser dabryanus dumeril, vacuum filtration is performed by using a 0.22 µm mixed cellulose filter membrane, and thus a mixed cellulose filter membrane containing a water body DNA sample is obtained.
(2) The DNA sample on the filter membrane obtained in step (1) is extracted by using the E.Z.N.A.^{®}Water DNA Kit, a concentration of the DNA sample is tested by using the qubit, an initial concentration is determined as 6ng/µL, and preservation is performed at -20°C for standby use.
(3) The DNA sample with the initial concentration being 6ng/µL obtained in step (2) is diluted with ultrapure water respectively by 10 times, 100 times and 1000 times and used as a DNA template of ddPCR, and ultrapure water is used as a DNA template of negative control.
(4) a ddPCR amplification system is prepared by using the DNA template obtained in step (3), and then a Bio-Rad QX200TM droplet digital PCR instrument is used for testing, wherein the ddPCR amplification system is shown in Table 2, an amplification procedure is shown in Table 3, a testing result is shown in Table 4, and 3 parallel tests are repeated for each DNA template.

**Table 2 ddPCR amplification system**

| Reagent | Use amount |
|---|---|
| DNA Template | 8.5 µL |
| ND5-F4 | 0.5 µL |
| ND5-R4 | 0.5 µL |
| ND5 Probe4 | 0.5 µL |
| 2×ddPCR master mix | 10 µL |
| Total | 20 µL |

**Table 3 ddPCR amplification procedure**

| Temperature | Time | The number of cycles |
|---|---|---|
| 95°C | 10 min | 1 |
| 94°C | 30 s | 40 |
| 60°C | 40 s | |
| 72°C | 30 s | |
| | | |
| 98°C | 10 min | 1 |
| 4°C | ∞ | |

**Table 4 ddPCR testing result**

| | ND5 Probe4 testing concentration (copies/µL) |
|---|---|
| Acipenser dabryanus dumeril water sample DNA*10-¹ | 1428 |
| Acipenser dabryanus dumeril water sample DNA*10⁻² | 145 |
| Acipenser dabryanus dumeril water sample DNA*10⁻³ | 15.8 |
| Negative control | 0 |
| Acipenser dabryanus dumeril fin ray DNA | Exceed a testing range |

The result is shown in Table 4 that by using ND5-F4 and ND5-R4 as the pair of primers and using ND5 Probe4 as the Taqman fluorescent probe, the DNA concentration of the acipenser dabryanus dumeril may be successfully tested from the aquatic water body; and after 10-times dilution, the DNA testing concentration of the sample and the dilution multiple are reduced at the same proportion, and thus it proves effectiveness and stability of the pair of primers and the probe.

### Example 3 Sensitivity test of the pair of primers and the probe

The sensitivity test of the screened pair of specific primers ND5-F4/ND5-R4 and Taqman fluorescent probe ND5 Probe4 of acipenser dabryanus dumeril includes the following steps.
(1) To-be-tested water sample DNA of the acipenser dabryanus dumeril is diluted by using a double dilution method, the first dilution reaches 1/2 of an initial concentration, and the second dilution reaches 1/4 of the initial concentration, and so on to generate 10 types of diluent.
(2) The 10 types of diluent obtained in step (1) are used as the DNA template, the Bio-Rad QX200TM droplet digital PCR instrument is used for testing to obtain the testing concentration, and a testing method is the same as Example 2.
(3) Logarithmic transformation is performed on the testing concentration obtained in step (2), a correlation analysis is performed, and a correlation curve is drawn.

The dilution multiple and the sample ddPCR testing concentration are shown in Table 5: wherein the ddPCR testing concentration of the to-be-tested water sample is 112.2copies/µL, a minimum testing concentration is 0.05copies/µL after being diluted to 1024 times, and no DNA is detected when the concentration is diluted to 2048 times.

**Table 5 ddPCR testing result of gradiently diluted water sample DNA**

| The number of times of dilution | Gradient dilution multiple | ddPCR concentration (copies/µL) |
|---|---|---|
| 0 | 1 | 112.2 |
| 1 | 2 | 58.6 |
| 2 | 4 | 28 |
| 3 | 8 | 13.9 |
| 4 | 16 | 7 |
| 5 | 32 | 3.3 |
| 6 | 64 | 2.5 |
| 7 | 128 | 0.43 |
| 8 | 256 | 0.22 |
| 9 | 512 | 0.14 |
| 10 | 1024 | *0.05* |

The dilution multiple and the ddPCR testing concentration are subjected to logarithmic transformation and then may be in a linear relation, and a result is shown in Fig. 2 that it is (R²=0.989, P<0.01). To sum up, the pair of primers and probe have high test sensitivity for a water body sample of acipenser dabryanus dumeril, and the concentration of 0.05copies/µL or above can be detected through ddPCR.

### Example 4 Tracking of a behavior trajectory of acipenser dabryanus dumeril released

On October 24, 2021, opposite to an estuary of the Minjiang River (between section 12 and section 13), about 35000 pieces of acipenser dabryanus dumeril are released, and the behavior trajectory of the artificially released acipenser dabryanus dumeril is tracked by using the pair of specific primers ND5-F4/ND5-R4 and the Taqman fluorescent probe ND5 Probe4 thereof obtained through screening in the above examples, which specifically includes the following steps.
(1) Through literature research, reaches of downstream of the Jinsha River and upstream of the Yangtze River basin where the acipenser dabryanus dumeril is centralized historically are: from a position under Xiangjiaba dam to Lizhuang reach, a section is set every 2 km to 3 km in this reach (Table5), and three sampling points on left, middle and right are set on each section.
(2) 2L of a water body is collected from the sampling points set in step (1) according to release time (on October 24, 2021) for the acipenser dabryanus dumeril as a sample; wherein the sampling time is one day before release (on October 23), and five days after release (from October 25 to October 29).

**Table 5 Longitude and latitude of a section**

| Section | Northern latitude N | East longitude E | Section | Northern latitude N | East longitude E |
|---|---|---|---|---|---|
| 1 | 28.6444 | 104.408315 | 12 | 28.76031 | 104.618381 |
| 2 | 28.628447 | 104.423309 | MJ | 28.777335 | 104.624194 |
| 3 | 28.629519 | 104.446731 | 13 | 28.771227 | 104.647474 |
| 4 | 28.640867 | 104.465111 | 14 | 28.763563 | 104.670436 |
| 5 | 28.651458 | 104.482549 | 15 | 28.773577 | 104.684309 |
| 6 | 28.674059 | 104.494649 | 16 | 28.785697 | 104.698265 |
| 7 | 28.685015 | 104.512583 | 17 | 28.778944 | 104.723498 |
| 8 | 28.689152 | 104.536334 | 18 | 28.774103 | 104.741637 |
| 9 | 28.701061 | 104.556898 | 19 | 28.783684 | 104.754974 |
| 10 | 28.714637 | 104.581713 | 20 | 28.788853 | 104.768238 |
| 11 | 28.73992 | 104.593057 | 21 | 28.810102 | 104.796046 |

| | | | | | |
|---|---|---|---|---|---|
| Notes: MJ is a release position. | | | | | |

(3) The collected water sample is made to pass through the mixed cellulose filter membrane having a hole diameter of 0.22 µm within 24 h by using a vacuum filtration method, and the filtered mixed cellulose filter membrane is put in a centrifugal tube to be preserved at -20°C, wherein one sheet of filter membrane is used for each liter of the water body, gloves are worn during the entire operation process, the filter membrane is transferred by using a pair of tweezers, and a filter is flushed with sterile water before filtration of samples in different sections to avoid cross contamination.
(4) The E.Z.N.A.Water DNA Kit is used for extracting DNA of the mixed cellulose filter membrane containing water body DNA of each section and each sampling point obtained in step (3), and the DNA is preserved at -20°C, wherein the ultrapure water is used as a DNA template for negative control.
(5) ddPCR is performed by using the pair of specific primers ND5-F4/ND5-R4 and the Taqman fluorescent probe ND5 Probe4 determined in Example 3 for testing a concentration of a target fragment in each sample.

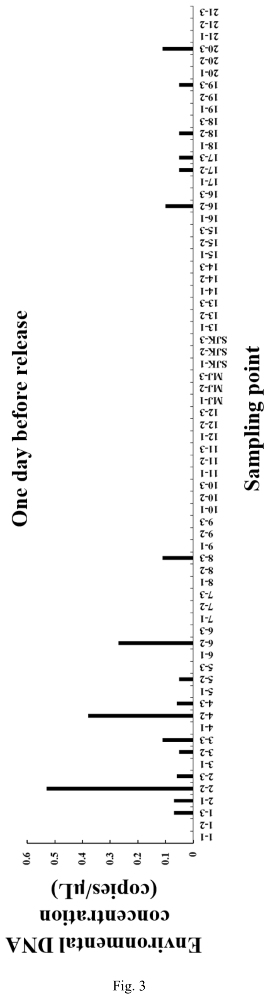

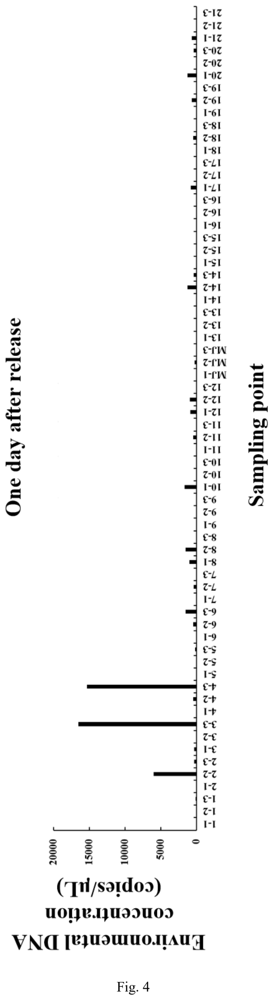

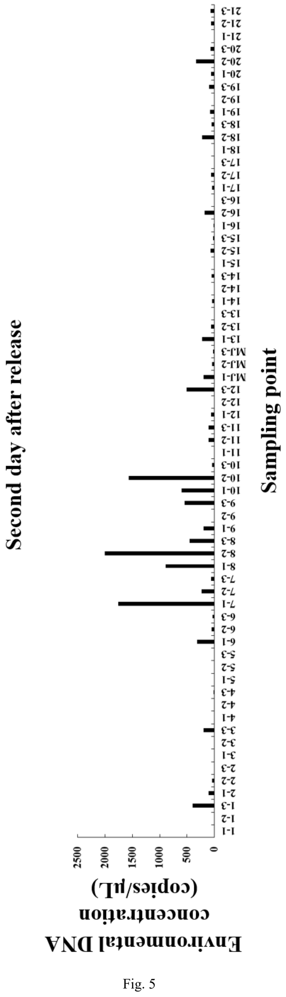

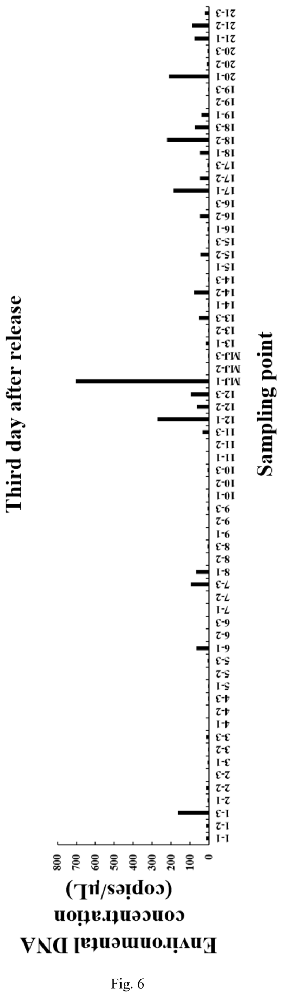

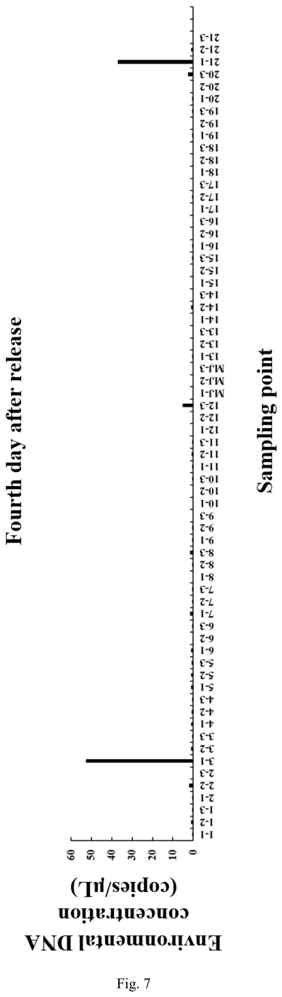

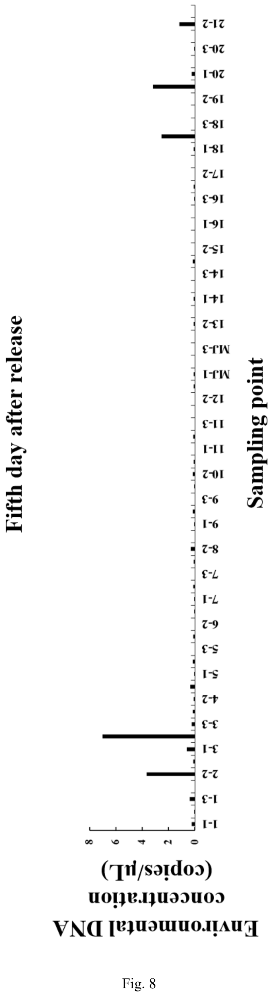

It can be seen from Fig. 3 to Fig. 8 that the concentration of the environmental DNA of the acipenser dabryanus dumeril before release is far lower than a concentration after release, and a relatively high concentration mainly appears within 10 km range under the Xiangjiaba dam. A sampling result on the first day (October 25) after release shows that concentrations of the first four sections are high, the released acipenser dabryanus dumeril is mainly distributed within the 10 km range from the Xiangjiaba dam to downstream; and environmental DNA samples having higher concentrations on the second day (October 26) after release are centralized in section 7 to section 10. On the third day (October 27) after release, environmental DNA samples having high concentrations are mainly distributed in section 12, the estuary of the Minjiang river, and sections 18 to 20, which indicates that the released acipenser dabryanus dumeril is mainly distributed in sections 12 to 20. On the fourth day (October 28) after release, except section 3 and section 21, the other sections have the concentration values close to those before release. On the fifth day (October 29) after release, the tested environmental DNA testing concentration and centralized distribution range of the acipenser dabryanus dumeril are similar to those before release. It can be seen according to the testing result that the released acipenser dabryanus dumeril has an obvious motion tendency, and a summary for the motion tendency of the released acipenser dabryanus dumeril through sampling in this year is as follows.
1. Within a range from 5 km to 10km under the Xiangjiaba dam, a sample with a relatively high concentration may be collected before and after release, and it may be deduced that acipenser dabryanus dumeril has always existed here.
2. After releasing the acipenser dabryanus dumeril, the water body environmental DNA concentration of the acipenser dabryanus dumeril surges, and after five days, the environmental DNA concentration value is close to a concentration value before release. It can be known according to the tested concentration change tendency that at an initial stage of release, an activity range of the acipenser dabryanus dumeril is relatively centralized, a distribution of the acipenser dabryanus dumeril extended over time, and thus a concentration value obtained from a fixed sampling amount shows a constant decrease tendency.
3. It can be known according to an environmental DNA concentration testing result five days after release and a release point position (between section 12 and section 13) that after releasing from the release point, most of acipenser dabryanus dumeril moves upwards first towards the Xiangjiaba dam and then starts to move downwards.

### Example 5 Sonar monitoring for a behavior trajectory of acipenser dabryanus dumeril released

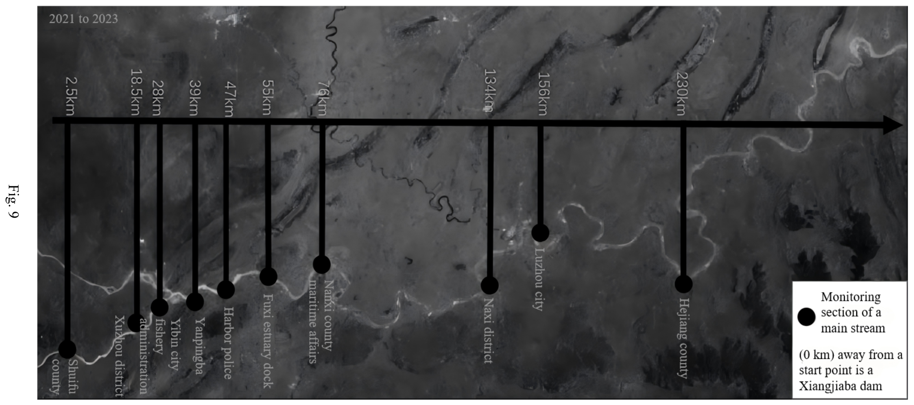

For proving the probe method-based testing result of the environmental DNA in Example 4, on November 12 in the same year, 26 pieces of V7 and V16 sonar marked juvenile acipenser dabryanus dumeril are released in the Shuifu county, and the behavior trajectory of the acipenser dabryanus dumeril after release is tested through a plurality of sonar signal fixed monitoring stations (Fig. 9).

A result is shown in Fig. 10 that the 26 pieces of marked juvenile acipenser dabryanus dumeril successfully receive a total of sonar signals of 22 pieces of juvenile acipenser dabryanus dumeril. From after release to April, 2022 (in April next year), juvenile acipenser dabryanus dumeril released is mainly centralized from under the dam to a reach of Nanxi County maritime affairs to move (a correspondence between a water sample collection section and a sonar monitoring section is: section 1 is about located in a position of the Shuifu county, section 12 is about located in a position of Yibin city fishery administration, a Yanpingba is located between section 15 and section 16, and section 19 is about located in a position of harbor police), wherein the reach of the Shuifu county has the largest number of times of obtaining signals and the longest duration of stay of juvenile fish, which indicates that the acipenser dabryanus dumeril has always been active in the reach of the Shuifu county (namely, the reach under the dam), and it is consistent with a result deduced by environmental DNA monitoring that the acipenser dabryanus dumeril has always been distributed under the dam.

Though the sonar monitoring method and the environmental DNA monitoring method have inconsistent accuracy, sonar monitoring cannot be as sensitive as the environmental DNA, and the environmental DNA cannot achieve long-time tracking for the acipenser dabryanus dumeril like sonar, the motion tendency of the acipenser dabryanus dumeril can be judged by comparing results of the two monitoring methods. After marking releasing on November 12, on the first two days (on November 13 and 14), signals mainly appear nearby the Shuifu county, and signals mainly appear in the Shuifu county and secondly at a Fuxi estuary on November 16 and 17, which is fully consistent with the result that on the first day after release, the acipenser dabryanus dumeril is mainly distributed in the reach under the dam and after five days, the acipenser dabryanus dumeril is mainly distributed in the reach under the dam and sections 18 to 21 (about 45 km to 65 km away from under the dam) monitored by environmental DNA. The number and frequency of received sonar signals are in a decrease tendency over time, which is consistent with the tendency that the monitored environmental DNA concentration gradually decreases, and it indicates that after release, juvenile fish may remain a relatively centralized distribution mode at first and later gradually spread.

The two monitoring means may mutually prove that the released acipenser dabryanus dumeril has always been distributed in the reach of Shuifu county under the dam; and at an initial stage of release, the acipenser dabryanus dumeril has a relatively centralized activity range, and the distribution range of the acipenser dabryanus dumeril extends over time. The release site of the sonar marked acipenser dabryanus dumeril this year is just located in Shuifu county under the dam, so a conclusion cannot be obtained from the testing result of Fig. 10 that the released acipenser dabryanus dumeril firstly moves upwards towards the Xiangjiaba dam and then moves downwards. However, according to the acipenser dabryanus dumeril marked and released between the Shuifu county and the Xuzhou district in 2020, it can be known according to a sonar tracking result (Fig. 11) that after releasing on October 24, signals of nine pieces of juvenile fish in successfully received signals of 25 pieces of juvenile fish firstly appear in Shuifu county under the dam and then are monitored in a downstream station, which proves the motion tendency of part of juvenile fish that firstly moves upwards and then downwards monitored by environmental DNA.

## Claims

1. A probe method-based kit for tracking a behavior trajectory of acipenser dabryanus dumeril released upstream of Yangtze river, wherein the kit comprises a pair of specific primers, a Taqman fluorescent probe, a DNA template, a ddPCR master mix and ddH₂O.

2. The probe method-based kit for tracking the behavior trajectory of acipenser dabryanus dumeril released upstream of Yangtze river according to claim 1, wherein the pair of specific primers comprises an upstream primer ND5-F4 with a sequence of SEQ ID NO: 1; and a downstream primer ND5-R4 with a sequence of SEQ ID NO:2; and the Taqman fluorescent probe has a sequence of SEQ ID NO:3.

3. The probe method-based kit for tracking the behavior trajectory of acipenser dabryanus dumeril released upstream of Yangtze river according to claim 1, wherein a 5' terminal of the Taqman fluorescent probe is connected with a fluorophore FAM, and a 3' terminal is connected with a quencher BHQ.

4. The probe method-based kit for tracking the behavior trajectory of acipenser dabryanus dumeril released upstream of Yangtze river according to claim 1, wherein the ddPCR master mix comprises 2×supermix.

5. The probe method-based kit for tracking the behavior trajectory of acipenser dabryanus dumeril released upstream of Yangtze river according to claim 1, wherein the DNA template is a water body sample DNA, and the sample comprises acipenser dabryanus dumeril DNA concentration ≥0.05copies/µL.

6. A probe method-based method for tracking a behavior trajectory of acipenser dabryanus dumeril released upstream of Yangtze river, wherein the method comprises the following steps:
(1) setting sampling sections in a researched water area, setting sampling points on the sampling sections, and collecting 2 L of water body on each sampling point every day one day before release and within five days after release;
(2) performing vacuum filtration on the collected water body within 24 h by using a mixed cellulose filter membrane, and preserving the mixed cellulose filter membrane obtained after filtration;
(3) extracting DNA on the mixed cellulose filter membrane and preserving the DNA at -20°C for standby use; and
(4) using the DNA obtained in step (3) as a template, carrying out ddPCR testing by using the tracking kit according to any one of claims 1 to 3, determining a concentration of a target sequence in each collected water body, and thus determining the behavior trajectory of the acipenser dabryanus dumeril.

7. The probe method-based method for tracking the behavior trajectory of acipenser dabryanus dumeril released upstream of Yangtze river according to claim 6, wherein in step (1), spacing between the sampling sections is 2 km to 3 km, and the number of sampling points is 2 to 5.

8. The probe method-based method for tracking the behavior trajectory of acipenser dabryanus dumeril released upstream of Yangtze river according to claim 6, wherein in step (2), the mixed cellulose filter membrane has a pore diameter of 0.22 µm.

9. The probe method-based method for tracking the behavior trajectory of acipenser dabryanus dumeril released upstream of Yangtze river according to claim 6, wherein in step (2), the preserving method is to preserve at -20°C or preserve in a Longmire's buffer at a room temperature.

10. The probe method-based method for tracking the behavior trajectory of acipenser dabryanus dumeril released upstream of Yangtze river according to claim 6, wherein in step (3), the method for extracting the DNA is to extract by using a Water DNA Kit.

11. The probe method-based method for tracking the behavior trajectory of acipenser dabryanus dumeril released upstream of Yangtze river according to claim 6, wherein in step (4), an amplification system of the ddPCR testing is 8.5 µL of the DNA template, 0.5 µL of a reverse primer and 0.5 µL of a forward primer, 0.5 µL of the probe, and 10 µL of 2×ddPCR master mix; and an amplification procedure is initial denaturation for 10 min at 95°C; denaturation for 30s at 94°C, annealing for 40s at 60°C, extension for 30s at 72°C, and 40 cycles; and enzyme inactivation for 10 min at 98°C, and maintaining at 4°C.
